# EUROPEAN PATENT SPECIFICATION

(11) **EP 3 970 768 B1**
(45) Date of publication and mention of the grant of the patent: **10.05.2023**
(21) Application number: 21194042.4
(22) Date of filing: 31.08.2021
(51) Int. Cl.: A61M 5/145, A61M 5/168, A61M 5/142

(54) **INFUSION PUMP WITH TOGGLING CAPABILITY**
INFUSIONSPUMPE MIT UMSCHALTMÖGLICHKEIT
POMPE DE PERFUSION AVEC CAPACITÉ DE VA-ET-VIENT

(30) Priority: 08.09.2020 US 202063075341 P
(43) Date of publication of application: 23.03.2022
(73) Proprietor: Eitan Medical Ltd., 4250529 Netanya (IL)
(72) Inventor: Rasowsky, Amir, Yakir (IL); Pesach, Gidi, Kfar Vitkin (IL); Shtoltz, Ram, Givatayim (IL); Eitan, Shaul, Hofit 4029500 (IL); Eitan, Boaz, Hofit 4029500 (IL)
(74) Representative: Evens, Paul Jonathan

(56) References cited:
- EP-A1- 3 705 148
- US-A1- 2015 005 732
- US-A1- 2015 292 500

## Description

### CROSS-REFERENCE TO RELATED APPLICATIONS

The present application claims the priority of US 63/075,341 to Eitan et al, filed September 8, 2020, entitled, "Infusion pump with toggling capability".

### FIELD OF THE INVENTION

The present invention relates generally to the field of medical devices for providing fluid to a patient. More specifically, the present invention relates to infusion pumps configured to inhibit unintended bolus liquid flow and/or reverse flow by compensation for the descending and/or ascending of the pump's downstream valve.

### BACKGROUND

Pumps are often used in the medical industry for delivering fluids, e.g., drugs, or diagnostic fluids, to subjects. One type of medical pump is an infusion pump, used to infuse a fluid into a subject's circulatory system via infusion tubing or a cassette at high accuracy and often for a prolonged period of time. Some infusion pumps pump fluid through the infusion tubing by repeatedly pressing, i.e., squeezing, the tubing.

Occlusions downstream of the pump may occur during the operation of the pump, for example, as a result of a kink in the infusion tube, clamping of the infusion tube, or blood clots. In the event of an occlusion, pressure may build up within the infusion tubing upstream of the occlusion location.

Some known methods for reducing this built-up pressure within an infusion tube are to disconnect the infusion tube from the patient and spill some of the infusion fluid out to release the pressure, which results in a loss of some of the infusion fluid, or to run the pump backwards, which may result in an intake of blood from the patient into the infusion tube.

International Patent Application Publication WO 2020/178824 to Eitan et al. , published on 10th September 2020 which is after the priority date claimed in the present application, describes an infusion pump including a plunger that is configured to squeeze a section of an infusion tube. A proximal valve, proximal to the plunger, ascends to allow infusion fluid intake from a reservoir to the infusion tube and descends to inhibit infusion fluid intake from the reservoir to the infusion tube. A distal valve, distal to the plunger, ascends to allow infusion fluid flow past the distal valve, and descends to inhibit infusion fluid flow past the distal valve. A controller controls the plunger, proximal valve, and distal valve by initiating descending of the plunger concurrently with or prior to the ascending of the distal valve, such that the descending of the plunger compensates for suction produced by the ascending of the distal valve, thereby reducing backflow of fluid from the subject. Other applications are also described.

EP 3705148, published on 9th September 2020 which is after the priority date claimed in the present application, relates to an infusion pump including inlet and outlet valves and a squeezing element configured to squeeze a fluid line coupled to the infusion pump.

US 2015/0292500 relates to a method for operating a peristaltic pump, and discloses apparatus according to the pre-amble of appended independent claim 1.

US 2015/0005732 relates to fluid line occlusion detection system and methods.

### SUMMARY OF THE INVENTION

In accordance with the present invention, there is provided apparatus according to appended independent claim 1. Embodiments of the present invention are defined in appended claims dependent on independent claim 1.

A problem of infusion pumps, addressed by some applications of the present disclosure, is an unwanted suction of a patient's blood as a result of an ascending of the pump's downstream valve and/or an undesired bolus being provided to the patient as a result of a descending of the pump's downstream valve.

In accordance with some applications of the present disclosure, the herein disclosed infusion pump advantageously includes a controller configured to control the descending/ascending of the plunger along with the ascending/descending of the pump's distal (e.g., downstream) valve, such that the flow caused by the ascending/descending of the pump's distal valve is compensated for by the plunger.

According to some arrangements, before or with opening the distal valve (also referred to herein as the downstream valve), the plunger is lowered, such that a volume of liquid, equal to the volume of blood that would otherwise be sucked into the infusion tube as a result of the ascending of the distal valve, is pumped towards the patient's open vein, thus counteracting/compensating for the unwanted backflow of blood.

Advantageously, such concurrent compensational movement of the plunger vis-à-vis the distal valve ensures that the flow rate of liquid into the patient is essentially devoid of spikes.

According to some arrangements, the valve may ascend and descend at a velocity that is correlated with a set flow rate. This may advantageously ensure that the volume pushed towards the patient by the descent of the downstream valve as well as the volume sucked by the ascent of the downstream valve do not momentarily alter the flow rate.

According to some arrangements, the upper position of the downstream/distal valve may be set such that while in its upper position the distal valve still squeezes the infusion tube in order to reduce boluses caused by the descending of the downstream valve. Advantageously, the distal valve still squeezing the infusion tube while in its upper position also minimizes power consumption, and thus cost of use. Additionally, the distal valve still squeezing the infusion tube while in its upper position ensures that the delivery of the infusion fluid is at an essentially constant volume regardless of a potential degradation of the infusion tube as well as inhibiting or at least reducing tube degradation.

A further undesired effect that is addressed by some applications of the present invention is an unwanted bolus delivery of fluid to the patient after resolution of an occlusion downstream of the pump. Fluid flow to the patient stops in the event of an occlusion downstream of the pump. However, the pump generally continues to pump the fluid toward the patient, resulting in an increase in pressure within the fluid line upstream of the occlusion. The occlusion is detected once a threshold level of pressure is detected, at which point an occlusion alert is triggered and the pump typically stops. The occlusion is typically resolved, e.g., released, by the patient or a clinician. Were the built-up pressure not to be released prior to the occlusion being released, then upon resolution of the occlusion, the volume of the accumulated fluid between the pump and the occlusion site might be delivered to the patient in a short time, i.e., as an unintended bolus. For example, at a delivery flow rate of 1 mL/hr it may take up to 20 min (1200 sec) to reach the occlusion pressure threshold, e.g., 1.2 Bar. After release of the occlusion, it takes around two seconds for the bolus to reach the patient, i.e., the accumulated volume of fluid is delivered to the patient at a flow rate that is 600 times faster than the desired delivery flow rate.

Typically, the volume of the bolus would be smaller than the dose of fluid the patient would have otherwise received by the point in time at which the occlusion is resolved. This is due to the following two factors: (i) during the time it takes the pump to detect the occlusion (while the pump continues to run and the pressure is building up) and for the occlusion to then be resolved, the patient would have otherwise been receiving the fluid, and (ii) fluid flow into the infusion tube from the reservoir slows down as a result of backpressure caused by the occlusion. However, despite the volume of the fluid delivered in the bolus being smaller than what the patient was supposed to receive by that point in time, the flow rate at which the bolus would be delivered to the patient, were the built-up pressure not to be released, is significantly higher than the desired delivery flow rate.

Therefore, in accordance with some applications of the present invention, methods and apparatus are provided for toggling the upstream and downstream valves of a pump upon detection of an occlusion, in order to reduce the pressure built up in the infusion line due to the occlusion. An occlusion downstream of the downstream valve is detected by measuring pressure within the infusion tubing of the pump using a pressure sensor. Once an occlusion is detected, releasing the built-up pressure by toggling the valves typically is performed as follows:
- while the upstream valve remains closed, isolating a segment of the infusion tube between the upstream and downstream valves by closing the downstream valve,
- subsequently, while the downstream valve remains closed, reducing pressure in the isolated segment of the infusion tube by opening the upstream valve,
- subsequently, while the downstream valve remains closed, closing the upstream valve, and
- subsequently, while the upstream valve remains closed, reducing pressure downstream of the downstream valve, i.e., between the downstream valve and the occlusion, by opening the downstream valve.
Typically, the toggling is repeated a plurality of times until the pressure is released, at which point the pump alerts the user or clinician that there is an occlusion. The release of pressure by toggling the valves mitigates the delivery of a bolus of fluid when the occlusion is resolved.

There is provided, in accordance with some applications of the present disclosure, a method for use with an infusion pump coupled to a fluid reservoir, the infusion pump including an infusion tube, an upstream valve, a downstream valve, a pressing surface disposed between the upstream and downstream valves and configured to press on the infusion tube, and a pressure sensor, the method including:
measuring pressure within the infusion tube using the pressure sensor;
in response to the measured pressure, determining that there is an occlusion in the infusion tube downstream of the downstream valve; and
in response to the determination that there is an occlusion, toggling the upstream valve and the downstream valve, the toggling including:
   (a) while the upstream valve remains closed, isolating a segment of the infusion tube between the upstream and downstream valves by closing the downstream valve;
   (b) subsequently, while the downstream valve remains closed, reducing pressure in the isolated segment of the infusion tube by opening the upstream valve;
   (c) subsequently, while the downstream valve remains closed, closing the upstream valve; and
   (d) subsequently, while the upstream valve remains closed, reducing pressure downstream of the downstream valve by opening the downstream valve.

For some applications, toggling includes toggling while the infusion tube is partially squeezed by the pressing surface.

For some applications, the pressure sensor is positioned between the upstream valve and the downstream valve.

For some applications, the method further includes:
withholding generating an alert indicative of the occlusion in response to determining that there is an occlusion in the infusion tubing; and
subsequently to the toggling, generating an alert indicating to a user of the infusion pump that there is an occlusion in the infusion tube.

For some applications, the method further includes repeating the toggling a plurality of times, thereby reducing pressure that is in the infusion tube due to the occlusion.

For some applications, the method further includes stopping the toggling after a predetermined number of toggling cycles.

For some applications, the method further includes setting the predetermined number of toggling cycles based on the measured pressure indicative of the occlusion.

For some applications, the method further includes stopping the toggling after a predetermined amount of time.

For some applications, the method further includes setting the predetermined amount of time based on the measured pressure indicative of the occlusion.

For some applications, the method further includes generating an alert indicating to a user of the infusion pump that there is an occlusion in the infusion tube, and repeating the toggling a plurality of times includes repeating the toggling a plurality of times prior to generating the alert.

For some applications, repeating the toggling a plurality of times includes repeating the toggling at least two times prior to generating the alert.

For some applications, repeating the toggling a plurality of times includes repeating the toggling at least five times prior to generating the alert.

For some applications, repeating the toggling a plurality of times includes repeating the toggling up to 100 times prior to generating the alert.

For some applications, repeating the toggling a plurality of times includes repeating the toggling up to 50 times prior to generating the alert.

For some applications, repeating the toggling a plurality of times includes repeating the toggling up to ten times prior to generating the alert.

For some applications, toggling further includes:
repeatedly measuring pressure in the infusion tube; and
regulating the toggling in response to the repeated measuring of the pressure.

For some applications, the method further includes stopping the toggling when at least a threshold pressure decrease, due to the toggling, is detected by the repeated measuring of the pressure.

For some applications, the method further includes stopping the toggling when a pressure value detected during the repeated measuring of the pressure passes below a threshold.

For some applications, the method further includes setting the threshold based on the measured pressure indicative of the occlusion.

For some applications, the method further includes, subsequently to the stopping of the toggling when the pressure value passes below the threshold:
assessing that the infusion tube is no longer occluded in response to detecting a further reduction in pressure in the infusion tube.

For some applications, stopping the toggling includes stopping the toggling based on an amount of time of the toggling, even if the pressure value detected during the repeated measuring of the pressure does not pass below the threshold.

For some applications, stopping the toggling includes stopping the toggling based on a number of toggling cycles, even if the pressure value detected during the repeated measuring of the pressure does not pass below the threshold.

For some applications, stopping the toggling includes stopping the toggling after fewer than 100 toggling cycles, even if the pressure value detected during the repeated measuring of the pressure does not pass below the threshold.

For some applications, stopping the toggling includes stopping the toggling after fewer than 50 toggling cycles, even if the pressure value detected during the repeated measuring of the pressure does not pass below the threshold.

There is provided, in accordance with some applications of the present invention, apparatus for use with an infusion tube and a fluid reservoir, the apparatus including:
an infusion pump configured to be coupled to the infusion tube and the fluid reservoir, the infusion pump including:
an upstream valve;
a downstream valve;
a pressing surface disposed between the upstream and downstream valves and configured to press on the infusion tube when the infusion pump is coupled to the infusion tube;
a pressure sensor configured to measure pressure within the infusion tube; and
a controller configured to:
   in response to the measured pressure, determine that there is an occlusion in the infusion tube downstream of the downstream valve,
characterised in that the controller is further configured to:
in response to the determination that there is an occlusion, toggle the upstream valve and the downstream valve, the toggling including:
(a) while the upstream valve remains closed, isolating a segment of the infusion tube between the upstream and downstream valves by closing the downstream valve;
(b) subsequently, while the downstream valve remains closed, reducing pressure in the isolated segment of the infusion tube by opening the upstream valve, the pressure being reduced by some infusion fluid flowing back toward the reservoir upon the opening of the upstream valve;
(c) subsequently, while the downstream valve remains closed, closing the upstream valve; and
(d) subsequently, while the upstream valve remains closed, reducing pressure downstream of the downstream valve by opening the downstream valve, the pressure being reduced by some of the infusion fluid flowing upstream from downstream of the downstream valve upon the opening of the downstream valve.

For some applications, the controller is configured to perform the toggling of the upstream valve and the downstream valve while the infusion tube is partially squeezed by the pressing surface.

For some applications, the pressure sensor is positioned between the upstream valve and the downstream valve.

For some applications, the controller is further configured to:
withhold generating an alert indicative of the occlusion in response to determining that there is an occlusion in the infusion tube; and
subsequently to the toggling, generate an alert indicating to a user of the infusion pump that there is an occlusion in the infusion tube.

For some applications, the controller is configured to repeat the toggling a plurality of times, thereby reducing pressure that is in the infusion tube due to the occlusion.

For some applications, the controller is configured to stop the toggling after a predetermined number of toggling cycles.

For some applications, the controller is further configured to set the predetermined number of toggling cycles based on the measured pressure indicative of the occlusion.

For some applications, the controller is configured to stop the toggling after a predetermined amount of time.

For some applications, the controller is further configured to set the predetermined amount of time based on the measured pressure indicative of the occlusion.

For some applications, the controller is further configured to generate an alert indicating to a user of the infusion pump that there is an occlusion in the infusion tube, and the controller is configured to repeat the toggling a plurality of times prior to generating the alert.

For some applications, the controller is configured to repeat the toggling at least two times prior to generating the alert.

For some applications, the controller is configured to repeat the toggling at least five times prior to generating the alert.

For some applications, the controller is configured to repeat the toggling up to 100 times prior to generating the alert.

For some applications, the controller is configured to repeat the toggling up to 50 times prior to generating the alert.

For some applications, the controller is configured to:
(a) using the pressure sensor, repeatedly measure the pressure in the infusion tube during the toggling, and
(b) regulate the toggling in response to the repeated measuring of the pressure.

For some applications, the controller is configured to stop toggling when at least a threshold pressure decrease, due to the toggling, is detected by the repeated measuring of the pressure.

For some applications, the controller is configured to stop the toggling when a pressure value detected during the repeated measuring of the pressure passes below a threshold.

For some applications, the controller is further configured to set the threshold based on the measured pressure indicative of the occlusion.

For some applications. the controller is further configured to, subsequently to stopping the toggling when the pressure passes below the threshold, assess that the infusion tube is no longer occluded in response to detecting a further reduction in pressure in the infusion tube.

For some applications, the controller is configured to stop the toggling based on an amount of time of the toggling, even if the pressure value detected during the repeated measuring of the pressure does not pass below the threshold.

For some applications, the controller is configured to stop the toggling based on a number of toggling cycles, even if the pressure value detected during the repeated measuring of the pressure does not pass below the threshold.

For some applications, the controller is configured to stop the toggling after fewer than 100 toggling cycles, even if the pressure value detected during the repeated measuring of the pressure does not pass below the threshold.

For some applications, the controller is configured to stop the toggling after fewer than 50 toggling cycles, even if the pressure value detected during the repeated measuring of the pressure does not pass below the threshold.

For some applications, the controller is configured to stop the toggling after fewer than ten toggling cycles, even if the pressure value detected during the repeated measuring of the pressure does not pass below the threshold.

The present invention will be more fully understood from the following detailed description of applications thereof, taken together with the drawings, in which:

### BRIEF DESCRIPTION OF THE DRAWINGS

Examples illustrative of embodiments are described below with reference to the figures attached hereto. In the figures, identical structures, elements or parts that appear in more than one figure are generally labeled with a same numeral in all the figures in which they appear. Alternatively, elements or parts that appear in more than one figure may be labeled with different numerals in the different figures in which they appear. Dimensions of components and features shown in the figures are generally chosen for convenience and clarity of presentation and are not necessarily shown in scale. The figures are listed below.
Fig. 1 schematically illustrates an infusion pump;
Figs. 2A-B are, combined, an illustrative flowchart for operating an infusion pump;
Figs. 3A-B are, combined, a schematic illustration of the pumping cycle of an infusion pump, according to some applications of the present invention;
Fig. 4 is a schematic illustration of an occlusion downstream of a downstream valve of the infusion pump, in accordance with some applications of the present invention;
Fig. 5A is an illustrative flow diagram depicting the steps of toggling the upstream and downstream valves of the infusion pump, in accordance with some applications of the present invention;
Fig. 5B is a flowchart depicting the process of detecting an occlusion and toggling of the upstream and downstream valves in order to release the built-up pressure due to the occlusion, in accordance with some applications of the present invention; and
Fig. 6 is a graph showing results from an experiment carried out by the inventors, in accordance with some applications of the present invention.

### DETAILED DESCRIPTION

In the following description, various aspects of the disclosure will be described. For the purpose of explanation, specific configurations and details are set forth in order to provide a thorough understanding of the different aspects of the disclosure. However, it will also be apparent to one skilled in the art that the disclosure may be practiced without specific details being presented herein. Furthermore, well-known features may be omitted or simplified in order not to obscure the disclosure.

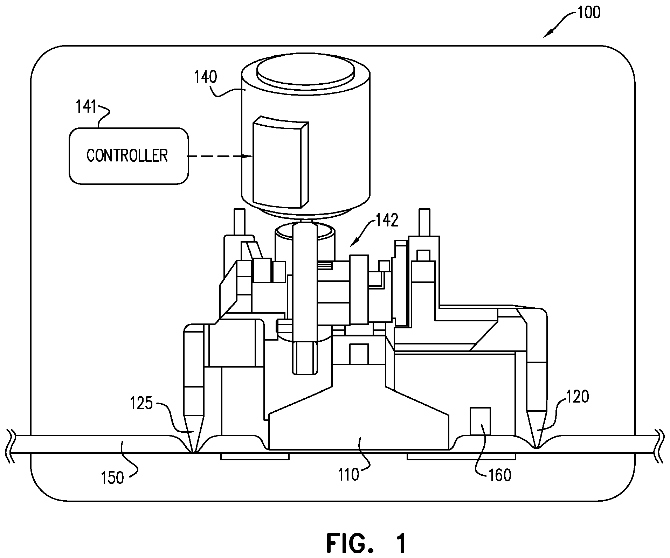

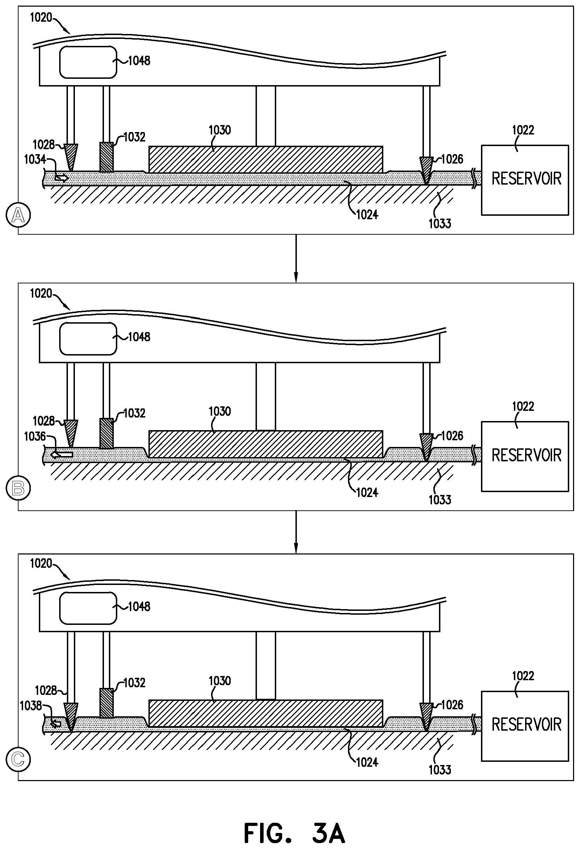

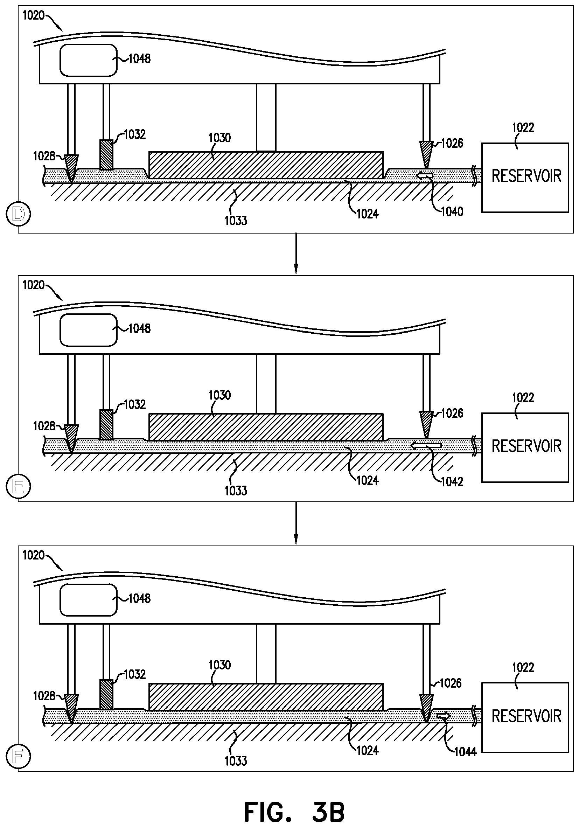

Reference is now made to FIG. 1 which schematically illustrates an infusion pump 100 comprising a plurality of fluid flow regulators. A plunger 110, e.g., a pressing surface such as pressing surface 1030 described hereinbelow, is one of the fluid flow regulators. Another one of the fluid flow regulators is a proximal/upstream valve 120, also referred to herein as an inlet valve, positioned proximally/upstream to plunger 110 and configured to allow flow of infusion fluid from a reservoir (e.g., reservoir 1022 as described hereinbelow with reference to Figs. 3A-B, 4, and 5A-B) to an infusion tube 150. A third one of the fluid flow regulators is a distal/downstream valve 125, also referred to herein as an outlet valve, positioned distally/downstream to plunger 110 and configured to allow flow of infusion fluid from infusion tube 150 to a patient (not shown). The positioning of plunger 110, proximal valve 120 and distal valve 125 are carried out by motor 140 and associated cam shaft 142, although other arrangements, according to which positioning of plunger 110, proximal valve 120 and distal valve 125 is executed by separate motors, are also possible and within the scope of this disclosure. Motor 140 is typically controlled by a controller 141. Infusion pump 100 further includes a force sensor 160 configured to measure the pressure in the part of infusion tube 150 extending between proximal valve 120 and distal valve 125. The figure illustrates the pump at a state when proximal valve 120 is at a tube releasing position, distal valve 125 is at a tube occluding position and plunger 110 is at a tube squeezing position.

According to some arrangements, there is provided infusion pump 100 comprising the plurality of fluid flow regulators as follows: a plunger configured to squeeze a section of an infusion tube; a proximal valve (also referred to herein as the "upstream valve") located proximally to the plunger; and a distal valve (also referred to herein as the "downstream valve") located distally to the plunger, wherein the distal valve is configured to ascend and thereby allow infusion fluid flow past the distal valve and to descend and thereby inhibit infusion fluid flow past the distal valve; and a controller configured to control the plunger, the proximal valve and the distal valve and thereby to control infusion fluid delivery to a subject and infusion fluid intake from an infusion source.

According to some arrangements, the infusion pump further comprises a pressure sensor. According to some arrangements, the controller is configured to control movement of the plunger based on pressure measurements obtained from the pressure sensor.

According to some arrangements, the controller is configured to move at least two of the fluid flow regulators in a synchronized manner to affect pressure within the infusion tube at least downstream of the downstream valve in order to prevent an undesired pressure condition downstream of the downstream valve. For example, according to some arrangements, the controller is configured to initiate an initial descending of the plunger prior to the ascending of the distal valve so as to create a positive pressure prior to the distal valve opening (while the proximal valve is closed) to compensate immediately for negative (i.e., upstream) fluid flow downstream of the downstream valve that would otherwise result from ascending of the distal valve. According to some arrangements, the controller is configured to initiate a partial initial ascending of the distal valve. According to some arrangements, a partial initial ascending of the distal valve may form a discrete fluid path opening in accordance with a set flow rate requirement. Advantageously such partial initial ascending of the distal valve may better couple the negative (upstream) and positive (downstream) infusion fluid flows and thus reduce boluses. According to some arrangements, the controller is configured to initiate a partial descending of the plunger concurrently with the continued ascending of the distal valve (while the proximal valve is closed). According to some arrangements, this partial descending of the plunger depends on pressure in the section of an infusion tube measured by the pressure sensor. According to some arrangements, the above-described descending of the plunger before or concurrently with the ascending of the distal valve may be referred to herein as a "compensatory" descending of the plunger that compensates for vacuum produced by the ascending of the distal valve, thereby reducing backflow of fluid from the subject. According to some arrangements, the descending of the plunger required for delivering the infusion fluid may be initiated once the distal valve reaches its upper position. As used herein, including in the claims, the term "upper position" of the distal valves refers to the maximum upper position of the distal valve in any given pump cycle, i.e., the upper limit of the distal valve stroke in any given pump cycle. According to some arrangements, the descending of the plunger required for delivering the infusion fluid may be initiated concurrently with the continued ascending of the distal valve to its upper position. According to some arrangements, this distal valve upper position may be dictated by pressure in the section of an infusion tube as measured by the pressure sensor.

According to some arrangements, the compensatory descending of the plunger may be performed separately from the descending of the plunger required for delivering the infusion fluid. According to some arrangements, the compensatory descending rate of the plunger may be same or different than the descending rate of the plunger required for delivering the infusion fluid. According to some arrangements, the compensatory descending of the plunger may be less than the descending of the plunger required for delivering the infusion fluid. According to some arrangements, the compensatory descending of the plunger may be coextensive with (an integral part of) the descending of the plunger required for delivering the infusion fluid.

Typically, the descending of the plunger being either (a) the compensatory descending of the plunger or (b) the descending of the plunger required for delivering the infusion fluid, depends on the state of the distal valve. While the distal valve is occluding the tube, the descending of the plunger is compensatory and acts to increase pressure in the isolated segment of the tube i.e., between the proximal and distal valves, thereby preparing a bolus that compensates for the suction that occurs when the distal valve is opened. For some set flow rates (e.g., less than 300 mL/h), the rate of the compensatory descending of the plunger is typically higher than the rate of the descending of the plunger required for delivering the infusion fluid.

The compensatory descending of the plunger is typically dependent on a predetermined volume of the bolus that compensates for the suction due to the ascending of the distal valve, which relates to parameters of the distal valve, e.g., the geometry of the distal valve, the stroke of the distal valve, and/or the size of the distal valve. Typically, the volume of the bolus prepared during the compensatory descending of the plunger is independent of the "set flow rate" of the infusion fluid (i.e., the flow rate which is set by an operator or programmer of the infusion pump). Contrary to the compensatory descending of the plunger, the descending of the plunger required for delivering the infusion fluid depends on the set flow rate of the infusion fluid.

According to some arrangements, the volume of the prepared bolus during the compensatory descending of the plunger may be calculated and accounted for as a part of the total delivered volume of infusion fluid. According to some arrangements, the compensatory descending of the plunger may be calculated according to pressure in the section of the infusion tube between the proximal and distal valves.

As used herein, the term "compensation" with regards to the plunger refers to a movement of the plunger which counteracts, nullifies, reverses, evens out or otherwise inhibits an undesired flow of infusion fluid to the patient's vein or reverse fluid/blood flow from the patient, caused by movement of the distal valve.

According to some arrangements, the distal valve is configured to ascend from a lower position, at which fluid delivery to the patient is essentially blocked, to an upper position, at which fluid delivery to the patient is facilitated. According to some arrangements, the ascending of the distal valve to the upper position may be minor such that the opening of the tube for delivery remains narrow (e.g., up to 30% area of the inner cross section of the infusion tube). Advantageously, at both the upper position and the lower position, the distal valve at least partially squeezes a section of the infusion tube, thereby reducing the volume of backflow caused by the ascending of the distal valve as well as enhancing the compensation for vacuum produced by the ascending of the distal valve and reducing power consumption. Advantageously, the smaller descending range of the valve reduces the positive flow bolus size.

According to some arrangements, descending of the plunger required for delivering the infusion fluid may be a descending of the plunger from an upper squeezing position to a lower squeezing position, wherein at both the upper squeezing position and the lower squeezing position, the plunger is squeezing a section of an infusion tube, such that an opposite side of an inner surface of the section does not contact the squeezed side, thus ensuring that the delivery of the infusion fluid is at an essentially constant volume regardless of a potential degradation of the infusion tube as well as inhibiting or at least reducing tube degradation.

As used herein, the term "infusion fluid" may refer to any fluid delivered to the patient such as, but not limited to, insulin, nutrients, saline solution, antibiotics, analgesics, anesthetics, hormones, vasoactive drugs, and chelation drugs, and any other therapeutic fluids or combination of fluids.

As used herein, the term "upper squeezing position" with regards to the plunger, refers to a position of a plunger at which an infusion tube is mildly squeezed (i.e., lower than a position at which the tube is not squeezed), without having the opposite sides of an inner surface of the squeezed section contacting one another. According to some arrangements, the delivery phase of the infusion pump is initiated at the "upper squeezing position" or at "after compensation" position. According to some arrangements, the upper squeezing position is higher (less squeezing of the tube) than the position of the plunger when descended to compensate for the backflow caused by the ascending of the distal valve.

As used herein, the term "lower squeezing position" with regards to the plunger, refers to a position of the plunger at which an infusion tube is squeezed to a larger extent as compared to the upper squeezing position, yet still without having the opposite sides of an inner surface of the squeezed section contacting one another.

As used herein, the term "degradation" may refer to the tube losing its springiness, becoming deformed, bottoming out, or otherwise changing its shape or consistency in a manner affecting the drug delivery accuracy. According to some arrangements, the infusion tube may be a DEHP-free PVC infusion tube, a DEHP containing infusion tube, a polyethylene (PE) tube, a silicone tube, a polyurethane tube or the like. Each possibility is a separate arrangement.

According to some arrangements, the velocity of the ascending and descending of the distal valve and/or of the plunger depends on the set flow rate of the infusion fluid. According to some arrangements, the ascending and/or descending of the plunger may be continuous, i.e., at a constant rate. According to some arrangements, the ascending and/or descending of the plunger may be pulsatory, i.e., in small steps. According to some arrangements, the ascending and/or descending of the distal valve may be continuous, i.e., at a constant rate. According to some arrangements, the ascending and/or descending of the distal valve may be pulsatory, i.e., in small steps. That is, at high set flow rates, the velocity of the ascending and descending of the distal valve and/or of the plunger may likewise be high, and at low set flow rates, the velocity of the ascending and descending of the distal valve and/or of the plunger may likewise be low. According to some arrangements, the controller may be configured to automatically adjust the velocity of the ascending and descending of the distal valve and/or of the plunger according to the set flow rate. This ascending/descending sequence and control of movement allow continued fluid delivery flow. For example, the controller may adjust the rate of the descending of the distal valve based on the set flow rate in order to avoid a bolus delivery upon descending of the distal valve, i.e., the rate of the descending of the distal valve is controlled by the controller such that as the distal valve descends the distal valve pushes infusion fluid to the subject at the set flow rate. Thus, for higher flow rates, i.e., faster descending of the plunger, a faster descending of the distal valve may be set, and for lower flow rates, i.e., slower descending of the plunger, a slower descending of the distal valve may be set. The extra volume of fluid pushed to the subject during the descending of the distal valve may be calculated and accounted for as part of the volume of infusion fluid delivered per pump cycle.

Typically, the "upper position" of the distal valve refers to a position of the distal valve at which an infusion tube is squeezed (i.e., lower than a position at which the tube is not squeezed), without having the opposite sides of an inner surface of the squeezed section contacting one another. That is, at the upper position the distal valve engages the infusion tube.

As used herein, the term "lower position" with reference to the distal valve refers to a position of the distal valve at which the infusion tube is squeezed to such extent that delivery of infusion fluid to the patient is essentially avoided.

According to some arrangements, the inner tube cross section of the infusion tube when the distal valve is in its upper position is 30%-98% (e.g., a preset value, e.g., 50%) of the area of the inner tube cross section of the infusion tube, when non-squeezed. Various upper positions for the distal valve may be used, affecting the percentage of the squeezed area, for different set flow rates. For example, while in its upper position the distal valve still squeezes the infusion tube in order to reduce the bolus that is caused by the descending of the distal valve. Nevertheless, while in its upper position the distal valve should be open enough so as not to inhibit delivery of the infusion fluid during the descending of the plunger.

According to some arrangements, for a typical tube of 3 mm inner diameter and a wall thickness of 0.5 mm, a typical upper position, of the distal valve is about 0.3 mm to 2.8 mm, lower than the diameter of a non-squeezed tube. Each possibility is a separate arrangement.

According to some arrangements, the infusion pump is configured to maintain an essentially constant flow rate during the entire delivery of an infusion fluid. As a non-limiting example, the infusion pump is configured to maintain a delivery of an infusion fluid at a set flow rate of 1± 0.05 mL/hour for at least 20, at least 36 or at least 96 hours.

According to some arrangements, the pump further includes a motor in communication with the controller, the motor configured to operate the plunger. According to some arrangements, the motor may further be configured to operate the proximal valve, the distal valve or both. Alternatively, the pump may include one or two additional motors configured to operate the proximal valve, the distal valve or both. According to some arrangements, the controller may control the operation of the motor, thereby determining the exact position of the plunger and/or the distal valve. According to some arrangements, the controller may control the operation of the motor, by determining a velocity/rate of the ascending/descending of the plunger, the proximal valve and/or the distal valve. According to some arrangements, the controller may control the operation of the motor, by determining the increments of the ascending/descending of the plunger, the proximal valve and/or the distal valve. According to some arrangements, the controller may control the operation of the motor, by determining the value of the ascending/descending of the plunger, the proximal valve and/or the distal valve.

According to some arrangements, the controller may further be configured to determine a "wait" period, during which the plunger remains at the upper squeezing position, thereby ensuring full engagement of the infusion tube with the plunger, prior to the closing of the infusion pump's upstream valve. This advantageously increases the accuracy of infusion fluid delivery in that the volume delivered remains constant even if the infusion tube has undergone degradation. Furthermore, due to the infusion tube fully engaging the plunger, a persistent ascending of the infusion tube after opening of the upstream valve is essentially inhibited. The length of the wait depends on the flow continuity requirements and the set flow rate. For low set flow rates (0.1-1 mL/hr) and flow continuity of bolus every 20 sec, the wait can last up to 18 sec. For higher set flow rates, the wait time can be shorter (e.g., about 10 sec) and for very high flow rates (999 mL/hr) it may last less than 1 second. The long wait is particularly advantageous for low set flow rates where the tube squeeze duty cycle is very long. The long wait times are essentially a no movement of plunger and valves in the specific position while for high set flow rates the "wait" equals pump's check-in time- the time the pump goes through the encoders that leave the plunger in the upper position while the proximal valve is open.

According to some arrangements, there is provided a method of operation of an infusion pump, the method comprising utilizing the infusion pump, as essentially described herein.

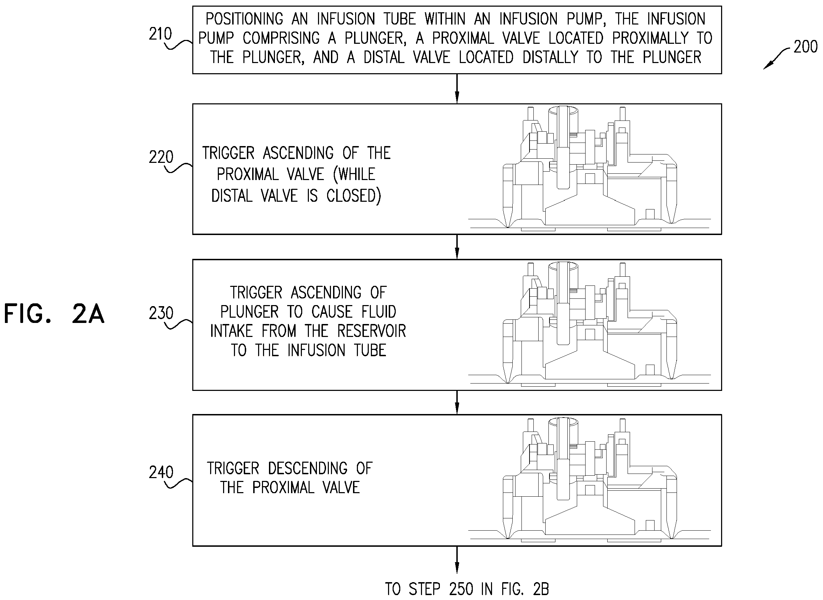

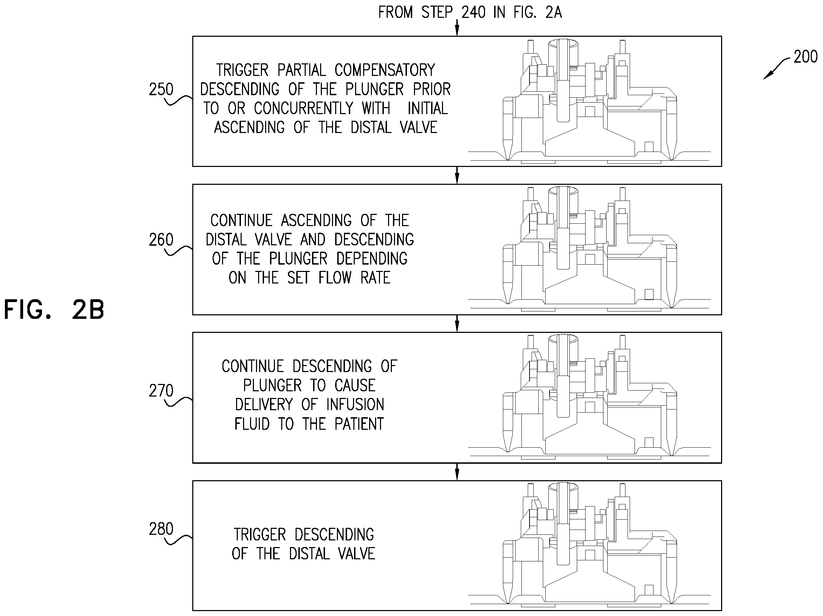

Reference is now made to FIGS. 2A-B which are, combined, an illustrative flowchart 200 for operating an infusion pump, according to some arrangements.

In step 210 an infusion tube is positioned within an infusion pump, the infusion pump comprising a plunger, a proximal valve located proximally to the plunger, and a distal valve located distally to the plunger.

Steps 220 to 240 are steps associated with intake of infusion fluid from a reservoir (also referred to herein as infusion source).

In step 220 an opening of the proximal valve is initiated (while the distal valve is closed), thereby establishing a fluid flow connection between the reservoir and the infusion tube.

In step 230 the plunger is caused to ascend, thereby causing intake of fluid from a reservoir. The ascending of the plunger, causing intake of the infusion fluid from the reservoir, is only initiated once the distal valve has reached its lower position at which the fluid flow connection between the infusion tube and the patient's vein has been closed.

In step 240 descending of the proximal valve is initiated to occlude the fluid line to terminate the fluid intake. The occlusion of the fluid line by proximal valve is completed before the distal valve starts ascending, thereby providing a phase where both valves are closed, during which the compensation step is carried out.

Steps 250 to 280 are steps associated with delivery of the infusion fluid to a patient.

In step 250 a partial, compensatory descending of the plunger is initiated prior to or concurrently with an initial ascending of the distal valve, thereby generating a positive pressure in the tube. Typically, up to 30% of the area of the inner tube cross section of the infusion tube is opened during the initial ascending motion of the distal valve. The compensatory descending of the plunger is configured to ensure that backflow of blood from the patient's vein into the infusion tube, as a result of the ascending of the distal valve, is reduced or inhibited. It is understood that the method alternatively may include two separate steps; a first step (e.g., step 250a) of partial compensatory descending of the plunger prior to the initial ascending of the distal valve and a second step (e.g., step 250b) of additional compensatory descending of the plunger concurrently with the ascending of the distal valve. During this step (250 or 250b), the pressure in the infusion tube between the valves is measured by the force sensor. If a decrease in pressure is observed, indicating that the distal valve has opened and downstream flow of infusion fluid is facilitated, the plunger transitions to descending at a rate corresponding to the set flow rate of the infusion fluid. It is noted that in the illustrative figure of step 250 the plunger and the distal valve appear to be in the same position as they are in the illustrative figure of step 240. The partial compensatory descending of the plunger is small and therefore not noticeably shown in the figure. Additionally, the distal valve is shown closed, since the partial compensatory descending of the plunger may occur prior to the initial ascending of the distal valve.

In step 260 the ascending of the distal valve and the descending of the plunger is continued. According to some arrangements, the rate of the ascending of the distal valve increases with the set flow rate, e.g., for higher set flow rates the distal valve ascends at a higher rate.

Optionally, in step 270 the plunger is further lowered thereby causing the infusion fluid to be delivered to the patient. Alternatively, the continuous ascending of the distal valve in step 260 may be very slow and prolonged, such that the delivery of the infusion fluid becomes an integral part of step 260.

In step 280, upon the plunger having completed the squeezing of the infusion tube, a descending of the distal valve is initiated to occlude the infusion line. The volume delivered due to descending of the distal valve may be determined and taken into account as part of the total volume of infusion fluid delivered, as described hereinabove. The rate of the descending of the distal valve may be adjusted to match the set flow rate of delivery.

It is understood that upon completion of infusion fluid delivery, additional intake/delivery cycles may be performed by repeating steps 220 through 280.

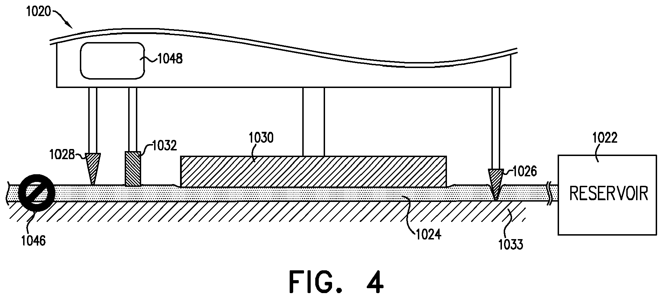

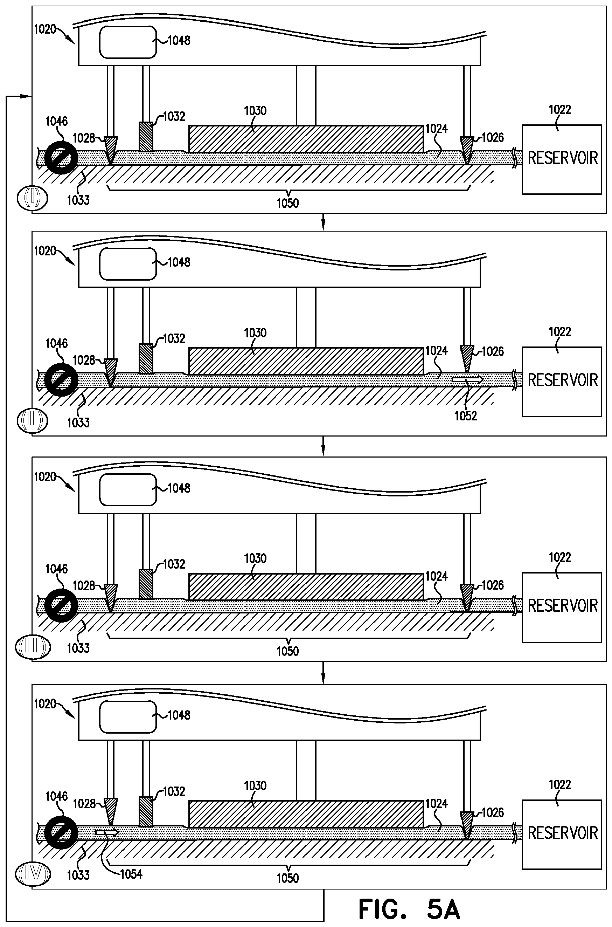

Reference is now made to Figs. 3A-B, which are illustrations of a pumping cycle of an infusion pump 1020. Infusion pump 1020 is coupled to a fluid reservoir 1022, e.g., an infusion bag or a syringe, and includes (i) an infusion tube 1024, (ii) a plurality of fluid flow regulators including an upstream valve 1026, a downstream valve 1028, and a pressing surface 1030, e.g., a plunger such as plunger 110 described hereinabove with reference to Fig. 1 (which is disposed between upstream valve 1026 and downstream valve 1028 and presses on infusion tube 1024), (iii) a pressure sensor 1032 configured to measure pressure within infusion tube 1024, and (iv) a controller 1048, e.g., a computer processor, further described hereinbelow with reference to Figs. 4 and 5A-B. According to some embodiments, controller 1048 is configured to move at least two of the fluid flow regulators (e.g., upstream valve 1026 and downstream valve 1028) in a synchronized manner (e.g., by toggling upstream valve 1026 and downstream valve 1028 as further described hereinbelow with reference to Figs. 5A-B) to affect pressure within the infusion tube at least downstream of the downstream valve in order to prevent an undesired pressure condition (e.g., an undesired bolus delivery due to built-up pressure) downstream of downstream valve 1028.

Figs. 3A-B depict steps A-F of the regular pumping cycle of infusion pump 1020. It is noted that while the pumping cycle is depicted as starting from the opening of downstream valve 1028 for delivery, this is not limiting and the pumping cycle may begin from any other point within the cycle, e.g., intake of fluid from reservoir 1022. It is also noted that for the sake of clarity of the illustration, each element in Figs. 3A-B is labeled only one time.

In step A, upstream valve 1026 is closed and downstream valve 1028 is opened for the start of delivery of the infusion fluid to the patient. Arrow 1034 illustrates that as downstream valve 1028 is opened a small amount of backflow may occur due to the opening of the valve. In step B, pressing surface 1030 is lowered in order to squeeze infusion tube 1024 and thereby cause the infusion fluid to flow toward the patient (not shown), as indicated by arrow 1036. In step C, once the delivery of the infusion fluid for that particular pumping cycle is complete, downstream valve 1028 is closed. Arrow 1038 illustrates that as downstream valve 1028 is closed a small amount of infusion fluid moves along infusion tube 1024 toward the patient due to the closing of the valve. For some applications, following step C, both upstream valve 1026 and downstream valve 1028 are maintained closed for a duration of time referred to herein as a "security zone" prior to the opening of upstream valve 1026, i.e., downstream valve 28 is closed prior to upstream valve 1026 being opened so as to prevent free flow of the infusion fluid to the patient upon the opening of upstream valve 1026.

In step D, the intake phase of the pumping cycle begins with the opening of upstream valve 1026 while downstream valve 1028 remains closed. Arrow 1040 illustrates that as upstream valve 1026 is opened a small amount of suction may occur due to the opening of the valve, causing a small amount of flow from reservoir 1022.

In step E, pressing surface 1030 is raised from the position it was lowered to in step B in order to create negative pressure within infusion tube 1024 to draw infusion fluid from reservoir 1022 into infusion tube 1024, as indicated by arrow 1042.

In step F, upstream valve 1026 is closed once the intake of infusion fluid from reservoir 1022 for that pumping cycle is complete. Arrow 1044 illustrates that as upstream valve 1026 is closed, a small amount of backflow may occur toward reservoir 1022 due to the closing of the valve. For some applications, following step F, both upstream valve 1026 and downstream valve 1028 are again maintained closed for a security zone, after which infusion pump 1020 returns to step A with the opening of downstream valve 1028.

Pressure sensor 1032 is positioned so as to measure pressure within infusion tube 1024. For some applications, pressure sensor 1032 may be any sensor configured to measure the pressure within infusion tube 1024, such as, but not limited to, a piezoelectric sensor, a gauge sensor, an optical sensor, a proximity sensor, or any combination thereof. For some applications, pressure sensor 1032 is positioned between upstream valve 1026 and downstream valve 1028.

Reference is now made to Fig. 4, which is a schematic illustration of an occlusion 1046 downstream of downstream valve 1028, in accordance with some applications of the present invention. For some applications, a controller 1048, e.g., a computer processor, is configured to, in response to the measured pressure within infusion tube 1024, determine that there is an occlusion 1046 in infusion tube 1024 downstream of the downstream valve 1028. For example, controller 1048 may determine that there is an occlusion 1046 in infusion tube 1024 in response to the measured pressure within infusion tube 1024 reaching an occlusion threshold value.

When occlusion 1046 occurs in infusion tube 1024 downstream of downstream valve 1028, the infusion fluid is prevented or at least inhibited from being delivered to the patient as pump 1020 continues to pump, and as such causes an increase in pressure within infusion tube 1024 between infusion pump 1020 and occlusion 1046. Typically, the pressure continues to increase until an occlusion threshold value is reached indicating that there is an occlusion within infusion tube 1024. As described hereinabove, were occlusion 1046 to be resolved prior to the built-up pressure being released, then the accumulated infusion fluid might be delivered to the patient as a bolus delivery at a flow rate that is typically substantially higher than the desired delivery flow rate, e.g., at least 5 times higher, e.g., at least 10 times higher, e.g., at least 50 times higher than the desired delivery flow rate. Therefore, in accordance with some applications of the present invention, in response to the determination that there is an occlusion 1046, controller 1048 toggles upstream valve 1026 and downstream valve 1028 in order to release the built-up pressure, as described hereinbelow with reference to Figs. 5A-B.

Reference is now made to Fig. 5A, which is an illustrative flow diagram depicting the steps of the toggling in order to release the built-up pressure in infusion tube 1024 due to the occlusion, in accordance with some applications of the present invention. The steps of the toggling are as follows:
Step (I): while upstream valve 1026 remains closed, isolating a segment 1050 of infusion tube 1024 between upstream valve 1026 and downstream valve 1028 by closing downstream valve 1028,
Step (II): subsequently, while downstream valve 1028 remains closed, reducing pressure in isolated segment 1050 of infusion tube 1024 by opening upstream valve 1026 (arrow 1052 illustrates that the pressure is reduced by some infusion fluid flowing back toward reservoir 1022 upon the opening of upstream valve 1026),
Step (III): subsequently, while downstream valve 1028 remains closed, closing upstream valve 1026, isolating segment 1050 again, and
Step (IV): subsequently, while upstream valve 1026 remains closed, reducing pressure downstream of downstream valve 1028 by opening downstream valve 1028 (arrow 1054 illustrates that the pressure is reduced by some of the infusion fluid flowing upstream from downstream of downstream valve 1028 upon the opening of downstream valve 1028; since some of the pressure was already reduced in step (II), arrow 1054 is depicted as slightly smaller than arrow 1052).

It is noted that an implementation is shown in Fig. 5A in which the toggling is performed while pressing surface 1030 is shown to be in its upper position, yet still slightly squeezing infusion tube 1024. Typically (but not necessarily), pressing surface 1030 is always in contact with infusion tube 24, even in its upper position (as shown in Figs. 3A-B as well). Typically, the toggling is performed without moving pressing surface 1030, such that the built-up pressure in infusion tube 1024 is passively released due to the toggling of upstream valve 1026 and downstream valve 1028. For some applications, the toggling is performed while pressing surface 1030 is maintained in whatever position it was in when the occlusion was detected, such that the toggling is performed without moving pressing surface 1030. For some applications, pressing surface 1030 is moved to its upper position for the toggling; in this case pressing surface is moved to its upper positioned when downstream valve 1028 is closed and upstream valve 1026 is open so as to avoid backflow of infusion fluid and/or blood from the patient.

It is noted that during the toggling, the opening of upstream valve 1026 is typically only performed while the downstream valve 1028 is closed, and the opening of downstream valve 1028 is typically only performed while upstream valve 1026 is closed. This is to prevent free-flow of the infusion fluid from reservoir 1022 to the patient in the event that the occlusion is released, e.g., accidentally released, during the toggling.

Arrow 1056 indicates that the toggling is iteratively repeated a plurality of times, further described hereinbelow.

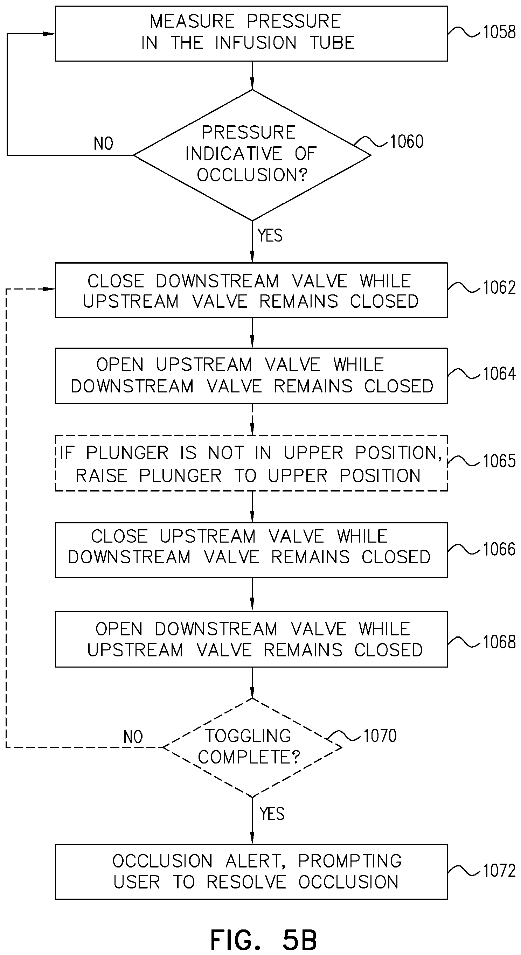

Reference is now made to Fig. 5B, which is a flowchart depicting the process of detecting an occlusion, e.g., occlusion 1046, in infusion tube 1024 and toggling of upstream valve 1026 and downstream valve 1028 in order to release the built-up pressure due to the occlusion, in accordance with some applications of the present invention. As described hereinabove, pressure is measured within infusion tube 1024 (step 1058) and controller 1048 determines that there is an occlusion downstream of the pump in response to the measured pressure, e.g., in response to the measured pressure reaching an occlusion threshold value. Decision diamond 1060 indicates that (a) if the measured pressure does not indicate an occlusion then pump 1020 continues to run while measuring the pressure within infusion tube 1024, and (b) if the pressure within infusion tube 1024 is indicative of an occlusion then the toggling of upstream valve 1026 and downstream valve 1028 (as described hereinabove with reference to Fig. 5A) is performed in order to release the built-up pressure. Steps (I), (II), (III), and (IV) of the toggling depicted in Fig. 5A correspond respectively to steps 1062, 1064, 1066, and 1068 of Fig. 5B.

For some applications, as indicated by decision diamond 1070, the toggling is iteratively repeated a plurality of times until controller 1048 determines that the toggling is complete. For some applications, controller 1048 stops the toggling, i.e., determines that the toggling is complete, after a predetermined number of toggling cycles, e.g., 10, 50, or 100 toggling cycles. Controller 1048 may set the predetermined number of toggling cycles in response to the measured pressure indicative of the occlusion. Alternatively or additionally, for some applications, the predetermined number of toggling cycles may be set based on (a) a length of infusion tube 1024, (b) a distance between upstream valve 1026 and downstream valve 1028, (c) the specific infusion fluid, e.g., based on a level of risk associated with deviation of the delivery rate for a specific fluid or medication, and/or (d) based on the delivery flow rate of the infusion.

With respect to the length of infusion tube 1024, the inventors have realized that as the length of infusion tube 1024 between occlusion 1046 and pump 1020 increases, it takes a higher number of toggling cycles to release the built-up pressure upstream of the occlusion. Therefore, for a long infusion tube 1024 where occlusion 1046 may be up to 2 m away from pump 1020, controller 1048 may set the predetermined number of toggling cycles to be higher than for a shorter infusion tube 1024.

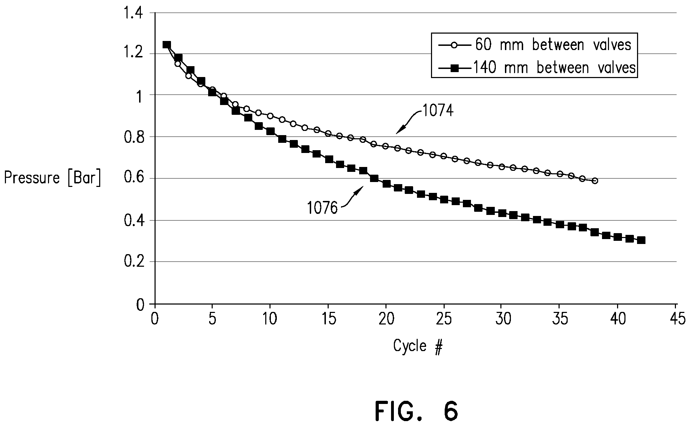

With respect to the distance between upstream valve 1026 and downstream valve 1028, the inventors have realized that as the length of isolated segment 1050, between upstream valve 1026 and downstream valve 1028, increases, it takes fewer toggling cycles to release the built-up pressure upstream of the occlusion. This is due to a higher amount of pressure being released per toggling cycle (further described with reference to Fig. 6). For example, a pump with a short isolated segment may require twice as many toggling cycles as a pump with an isolated segment that is twice the length.

With respect to the specific infusion fluid, for some applications, controller 1048 may have a setting that changes the pressure thresholds or the predetermined number of toggling cycles, or may entirely disable the toggling based on the specifics of a given clinical application. For example, for a particular drug in a particular clinical application it may be more desirable to give the entire missed volume in a single administration than to have the patient not receive any drug while the pressure is being released by the toggling, whereas for a different particular drug, e.g., a particularly potent drug, in a different particular clinical application, it may be harmful to the patient to give the entire missed volume in a single administration, in which case the toggling cycles are used to reduce the built-up pressure. Therefore, there is a tradeoff between whether an under-delivery or over-delivery is more desirable for a particular drug in the particular clinical application.

With respect to the delivery flow rate, controller 1048 may set the predetermined number of toggling cycles such that when occlusion 1046 is released the flow rate of the bolus delivery to the patient is relatively similar to the delivery flow rate. For example, with a high delivery flow rate, the flow rate of the potential unintended bolus (were the built-up pressure not to be released) may not be particularly different from the high delivery flow rate. In this case, controller 1048 may set the predetermined number of toggling cycles to be low or may disable the toggling feature completely. By contrast, with very low delivery flow rates, the flow rate of the potential unintended bolus (were the built-up pressure not to be released) would cause a high over-delivery rate with respect to the intended low delivery flow rate. This may be undesirable, e.g., for drugs having immediate effect on a patient's heart.

For some applications, controller 1048 stops the toggling, i.e., determines that the toggling is complete, after a predetermined amount of time, e.g., 0.5 - 15 seconds, e.g., 3 seconds. For some applications, the predetermined amount of time may be based on the measured pressure indicative of the occlusion, e.g., controller 1048 may set the predetermined amount of time in response to the measured pressure indicative of the occlusion. Alternatively or additionally, and as described hereinabove with regard to the predetermined number of toggling cycles, *mutatis mutandis,* for some applications, the predetermined amount of time may be set based on (a) a length of infusion tube 1024, (b) a distance between upstream valve 1026 and downstream valve 1028, (c) the specific infusion fluid, e.g., based on a level of risk associated with deviation of the delivery rate for a specific fluid or medication, and/or (d) based on the delivery flow rate of the infusion.

For some applications, using pressure sensor 1032, controller 1048 repeatedly measures the pressure in infusion tube 1024 during the toggling, and regulates the toggling in response to the repeated measuring of the pressure. For example, controller 1048 may stop the toggling, i.e., determine that the toggling is complete, when at least a threshold pressure decrease, due to the toggling, is detected by the repeated measuring of the pressure. Alternatively or additionally, controller 1048 may stop the toggling, i.e., determine that the toggling is complete, when a pressure value detected during the repeated measuring of the pressure passes below a threshold. For some applications, controller 1048 may set the threshold based on the measured pressure indicative of the occlusion.

For some applications, even if the pressure value detected during the repeated measuring of the pressure does not pass below the threshold, controller 1048 may stop the toggling based on (a) an amount of time of the toggling or (b) a number of toggling cycles, e.g., after fewer than 100 toggling cycles, e.g., after fewer than 50 toggling cycles, e.g., after fewer than 10 toggling cycles. For example, if after a predetermined amount of time or number of toggling cycles the pressure in infusion tube 1024 has still not decreased, then it may be an indication of something in pump 1020 not working correctly, e.g., pressure sensor 1032 not working, or one or both of upstream valve 1026 and downstream valve 1028 not working. Alternatively, if after a predetermined amount of time or number of toggling cycles the pressure in infusion tube 1024 has still not decreased, it may be due to another device having been connected to the same port on the patient's body and causing a continuous elevated pressure level which controller 1048 of pump 1020 may incorrectly interpret as an occlusion. In this case, continuous upstream flow may be caused if the toggling cycles were to continue indefinitely until a pressure decrease is detected. For some applications, controller 1048 may generate an alert if after a predetermined amount of time or number of toggling cycles the pressure in infusion tube 1024 has still not decreased, e.g., if the pressure remains at or above the threshold.

Step 1072 indicates that an occlusion alert is generated after the toggling is complete. As described hereinabove, toggling the upstream and downstream valves enables the built-up pressure from the occlusion to be released prior to the occlusion being resolved. Therefore, for some applications, even though controller 1048 has determined the presence of an occlusion in infusion tube 1024, an alert is not generated until after the toggling is complete. That is, the toggling is repeated a plurality of times, e.g., at least 2 times, e.g., at least 5 times, e.g., up to 100 times, e.g., up to 50 times, prior to generating the alert. Thus, for some applications, controller 1048 withholds generating an alert indicative of the occlusion until in response to determining that there is an occlusion, and subsequently to the toggling, the controller generates an alert indicating to a user of infusion pump 1020 that there is an occlusion in infusion tube 1024 (step 72). The user may then be prompted to resolve the occlusion.

For some applications, the valves are toggled until the built-up pressure due to the occlusion is largely or entirely released. However, it is possible that if the pressure is released and subsequently the occlusion is not actually resolved, e.g., due to human error, it may be a considerable amount of time before the pressure increases again to the occlusion pressure threshold, resulting in a long time without delivery of the fluid to the patient. Therefore, for some applications, the built-up pressure is reduced only to a point where a hazardous bolus would be avoided, e.g., the pressure threshold indicating that the toggling is complete may be above zero, e.g., 0.3 bar, and the remainder of the pressure is released by opening downstream valve 1028 after the occlusion has been resolved, e.g., after a clinician or user confirms resolution of the occlusion and/or has turned off the occlusion alert. Once resolution of the occlusion has been confirmed, controller 1048 opens downstream valve 1028 measures the pressure. Alternatively, subsequently to pump 1020 resuming delivery after resolution of an occlusion, controller 1048 may set the occlusion pressure level to be very low, e.g., zero bar, such that if the occlusion was not resolved and the pressure begins to increase again, pump 1020 will stop relatively quickly in order to reduce the pressure and alert the user again to the presence of the occlusion.

For some applications, setting the pressure threshold that indicates to controller 1048 that the toggling is complete to an above-zero level, e.g., 2 bar, enables controller 1048 to detect the resolution of the occlusion by detecting a further reduction in pressure in the infusion tube that occurs upon resolution of the occlusion. Thus, for some applications, subsequently to stopping the toggling when the pressure passes below the threshold, controller 1048 assesses that infusion tube 1024 is no longer occluded in response to detecting a further reduction in pressure in infusion tube 1024.

Reference is now made to Fig. 6, which is a graph showing results from an experiment carried out by the inventors, in accordance with some applications of the present invention. In the experiment an occlusion was artificially created by clamping infusion tube 1024 downstream of downstream valve 1028, creating approximately a 1.2 Bar pressure downstream of downstream valve 1028. An infusion bag was positioned 50 cm above upstream valve 1026 and downstream valve 1028. Toggling of upstream valve 1026 and downstream valve 1028 was performed as described hereinabove with reference to Figs. 5A-B. The experiment was carried out using two different pump geometries: (1) a Q-Core administration set (AS) (AP-404) with having a segment of 60 mm between upstream valve 1026 and downstream valve 1028 (curve 1074), and (2) a Fresenius AS tube with having a segment of 140 mm between upstream valve 1026 and downstream valve 1028 (curve 1076). Fig. 6 shows the pressure results obtained from the experiment. As seen from the graph, for both infusion tubes, the pressure decreased as a function of the number of toggling cycles. Additionally, as seen, the number of toggling cycles it takes to reduce the pressure to a given pressure level appears to depend on the distance between upstream valve 1026 and downstream valve 1028. When the distance between the valves is larger, the amount of infusion fluid flowing back toward the infusion bag when upstream valve 1026 is opened during the toggling is larger and thus fewer toggling cycles are needed to release the pressure built up in infusion tube 1024 due to the occlusion.

Applications of the invention described herein can take the form of a computer program product accessible from a computer-usable or computer-readable medium (e.g., a non-transitory computer-readable medium) providing program code for use by or in connection with a computer or any instruction execution system, such as controllers 141 and 1048. For the purpose of this description, a computer-usable or computer readable medium can be any apparatus that can comprise, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Typically, the computer-usable or computer readable medium is a non-transitory computer-usable or computer readable medium.

Examples of a computer-readable medium include a semiconductor or solid-state memory, magnetic tape, a removable computer diskette, a random-access memory (RAM), a read-only memory (ROM), a rigid magnetic disk and an optical disk. Current examples of optical disks include compact disk-read only memory (CD-ROM), compact disk-read/write (CD-R/W) and DVD. For some applications, cloud storage, and/or storage in a remote server is used.

A data processing system suitable for storing and/or executing program code will include at least one processor (e.g., controller 141, and controller 1048) coupled directly or indirectly to memory elements through a system bus. The memory elements can include local memory employed during actual execution of the program code, bulk storage, and cache memories which provide temporary storage of at least some program code in order to reduce the number of times code must be retrieved from bulk storage during execution. The system can read the inventive instructions on the program storage devices and follow these instructions to execute the methodology of the embodiments of the invention.

Network adapters may be coupled to the processor to enable the processor to become coupled to other processors or remote printers or storage devices through intervening private or public networks. Modems, cable modem and Ethernet cards are just a few of the currently available types of network adapters.

Computer program code for carrying out operations of the present invention may be written in any combination of one or more programming languages, including an object-oriented programming language such as Java, Smalltalk, C++ or the like and conventional procedural programming languages, such as the C programming language or similar programming languages.

It will be understood that the methods described herein can be implemented by computer program instructions. These computer program instructions may be provided to a processor of a general-purpose computer, special purpose computer, or other programmable data processing apparatus to produce a machine, such that the instructions, which execute via the processor of the computer (e.g., controller 141, and controller 1048) or other programmable data processing apparatus, create means for implementing the functions/acts specified in the methods described in the present application. These computer program instructions may also be stored in a computer-readable medium (e.g., a non-transitory computer-readable medium) that can direct a computer or other programmable data processing apparatus to function in a particular manner, such that the instructions stored in the computer-readable medium produce an article of manufacture including instruction means which implement the function/act specified in the methods described in the present application. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide processes for implementing the functions/acts specified in the methods described in the present application.

The controller (e.g., controller 141, and controller 1048) is typically a hardware device programmed with computer program instructions to produce a special purpose computer. For example, when programmed to perform the methods described herein, the controller typically acts as a special purpose computer processor. Typically, the operations described herein that are performed by computer processors transform the physical state of a memory, which is a real physical article, to have a different magnetic polarity, electrical charge, or the like depending on the technology of the memory that is used.

It will be appreciated by persons skilled in the art that the present invention is not limited to what has been particularly shown and described hereinabove. Rather, the scope of the present invention is defined by the appended claims, and may include both combinations and subcombinations of the various features described hereinabove, as well as variations and modifications thereof that are not in the prior art, which would occur to persons skilled in the art upon reading the foregoing description.

## Claims

1. Apparatus for use with an infusion tube (1024) and a fluid reservoir (1022), the apparatus comprising:
an infusion pump (1020) configured to be coupled to the infusion tube (1024) and the fluid reservoir (1022), the infusion pump (1020) comprising:
an upstream valve (1026);
a downstream valve (1028);
a pressing surface (1030) disposed between the upstream and downstream valves (1026,1028) and configured to press on the infusion tube (1024) when the infusion pump (1020) is coupled to the infusion tube (1024);
a pressure sensor (1032) configured to measure pressure within the infusion tube (1024); and
a controller (1048) configured to:
in response to the measured pressure, determine that there is an occlusion in the infusion tube (1024) downstream of the downstream valve (1028),
**characterised in that** the controller (1048) is further configured to:
in response to the determination that there is an occlusion, toggle the upstream valve (1026) and the downstream valve (1028), the toggling comprising:
(a) while the upstream valve (1026) remains closed, isolating a segment of the infusion tube (1024) between the upstream and downstream valves (1026,1028) by closing the downstream valve (1028);
(b) subsequently, while the downstream valve (1028) remains closed, reducing pressure in the isolated segment of the infusion tube (1024) by opening the upstream valve (1026), the pressure being reduced by some infusion fluid flowing back toward the reservoir upon the opening of the upstream valve (1026);
(c) subsequently, while the downstream valve (1028) remains closed, closing the upstream valve (1026); and
(d) subsequently, while the upstream valve (1026) remains closed, reducing pressure downstream of the downstream valve (1028) by opening the downstream valve (1028), the pressure being reduced by some of the infusion fluid flowing upstream from downstream of the downstream valve (1028) upon the opening of the downstream valve (1028).

2. The apparatus according to claim 1, wherein the controller (1048) is configured to perform the toggling of the upstream valve (1026) and the downstream valve (1028) while the infusion tube (1024) is partially squeezed by the pressing surface (1030).

3. The apparatus according to any one of claims 1-2, wherein the pressure sensor (1032) is positioned between the upstream valve (1026) and the downstream valve (1028).

4. The apparatus according to any one of claims 1-3, wherein the controller 91048) is further configured to:
withhold generating an alert indicative of the occlusion in response to determining that there is an occlusion in the infusion tube (1024); and
subsequently to the toggling, generate an alert indicating to a user of the infusion pump (1020) that there is an occlusion in the infusion tube (1024).

5. The apparatus according to any one of claims 1-4, wherein the controller (1048) is configured to repeat the toggling a plurality of times, thereby reducing pressure that is in the infusion tube (1024) due to the occlusion.

6. The apparatus according to claim 5, wherein the controller (1048) is configured to stop the toggling after a predetermined number of toggling cycles.

7. The apparatus according to claim 6, wherein the controller (1048) is further configured to set the predetermined number of toggling cycles based on the measured pressure indicative of the occlusion.

8. The apparatus according to claim 5, wherein the controller (1048) is configured to stop the toggling after a predetermined amount of time.

9. The apparatus according to claim 5, wherein the controller (1048) is further configured to generate an alert indicating to a user of the infusion pump (1020) that there is an occlusion in the infusion tube (1024), and wherein the controller (1048) is configured to repeat the toggling a plurality of times prior to generating the alert.

10. The apparatus according to claim 9, wherein the controller (1048) is configured to repeat the toggling at least two times prior to generating the alert.

11. The apparatus according to claim 5, wherein the controller (1048) is configured to:
using the pressure sensor (1032), repeatedly measure the pressure in the infusion tube (1024) during the toggling, and
regulate the toggling in response to the repeated measuring of the pressure.

12. The apparatus according to claim 11, wherein the controller (1048) is configured to stop toggling when at least a threshold pressure decrease, due to the toggling, is detected by the repeated measuring of the pressure.

13. The apparatus according to claim 11, wherein the controller (1048) is configured to stop the toggling when a pressure value detected during the repeated measuring of the pressure passes below a threshold.

14. The apparatus according to claim 13, wherein the controller (1048) is configured to stop the toggling based on an amount of time of the toggling, even if the pressure value detected during the repeated measuring of the pressure does not pass below the threshold.

15. The apparatus according to claim 13, wherein the controller (1048) is configured to stop the toggling after a number of toggling cycles, the number being less than 100, even if the pressure value detected during the repeated measuring of the pressure does not pass below the threshold.

## Patentansprüche

1. Vorrichtung zur Verwendung mit einem Infusionsschlauch (1024) und einem Fluidreservoir (1022), wobei die Vorrichtung Folgendes umfasst:
eine Infusionspumpe (1020), die konfiguriert ist, um an den Infusionsschlauch (1024) und das Fluidreservoir (1022) gekoppelt zu werden, wobei die Infusionspumpe (1020) Folgendes umfasst:
ein stromaufwärtiges Ventil (1026);
ein stromabwärtiges Ventil (1028);
eine Druckfläche (1030), die zwischen dem stromaufwärtigen und dem stromabwärtigen Ventil (1026,1028) angeordnet und konfiguriert ist, um auf den Infusionsschlauch (1024) zu drücken, wenn die Infusionspumpe (1020) an den Infusionsschlauch (1024) gekoppelt ist;
einen Drucksensor (1032), der konfiguriert ist, um Druck innerhalb des Infusionsschlauchs (1024) zu messen; und
eine Steuerung (1048), die für Folgendes konfiguriert ist:
als Reaktion auf den gemessenen Druck, Bestimmen, dass eine Okklusion in dem Infusionsschlauch (1024) stromabwärts des stromabwärtigen Ventils (1028) vorliegt,
**dadurch gekennzeichnet, dass** die Steuerung (1048) ferner für Folgendes konfiguriert ist:
als Reaktion auf die Bestimmung, dass eine Okklusion vorliegt, Umschalten des stromaufwärtigen Ventils (1026) und des stromabwärtigen Ventils (1028), wobei das Umschalten Folgendes umfasst:
(a) während das stromaufwärtige Ventil (1026) geschlossen bleibt, Isolieren eines Segments des Infusionsschlauchs (1024) zwischen dem stromaufwärtigen und dem stromabwärtigen Ventil (1026,1028) durch Schließen des stromabwärtigen Ventils (1028);
(b) anschließend, während das stromabwärtige Ventil (1028) geschlossen bleibt, Reduzieren von Druck in dem isolierten Segment des Infusionsschlauchs (1024) durch Öffnen des stromaufwärtigen Ventils (1026), wobei der Druck durch etwas Infusionsfluid reduziert wird, das beim Öffnen des stromaufwärtigen Ventils (1026) zu dem Reservoir zurückfließt;
(c) anschließend, während das stromabwärtige Ventil (1028) geschlossen bleibt, Schließen des stromaufwärtigen Ventils (1026); und
(d) anschließend, während das stromaufwärtige Ventil (1026) geschlossen bleibt, Reduzieren von Druck stromabwärts des stromabwärtigen Ventils (1028) durch Öffnen des stromabwärtigen Ventils (1028), wobei der Druck reduziert wird, indem etwas von dem Infusionsfluid stromaufwärts von stromabwärts des stromabwärtigen Ventils (1028) beim Öffnen des stromabwärtigen Ventils (1028) fließt.

2. Vorrichtung nach Anspruch 1, wobei die Steuerung (1048) konfiguriert ist, um das Umschalten des stromaufwärtigen Ventils (1026) und des stromabwärtigen Ventils (1028) durchzuführen, während der Infusionsschlauch (1024) teilweise durch die Druckfläche (1030) zusammengedrückt wird.

3. Vorrichtung nach einem der Ansprüche 1-2, wobei der Drucksensor (1032) zwischen dem stromaufwärtigen Ventil (1026) und dem stromabwärtigen Ventil (1028) positioniert ist.

4. Vorrichtung nach einem der Ansprüche 1-3, wobei die Steuerung 91048) ferner für Folgendes konfiguriert ist:
Unterdrücken des Erzeugens eines Alarms, der die Okklusion angibt, als Reaktion auf Bestimmen, dass eine Okklusion in dem Infusionsschlauch (1024) vorliegt; und
anschließend an das Umschalten, Erzeugen eines Alarms, der einem Benutzer der Infusionspumpe (1020) angibt, dass eine Okklusion in dem Infusionsschlauch (1024) vorliegt.

5. Vorrichtung nach einem der Ansprüche 1-4, wobei die Steuerung (1048) konfiguriert ist, um das Umschalten eine Vielzahl von Malen zu wiederholen, wodurch Druck reduziert wird, der in dem Infusionsschlauch (1024) aufgrund der Okklusion ist.

6. Vorrichtung nach Anspruch 5, wobei die Steuerung (1048) konfiguriert ist, um das Umschalten nach einer vorbestimmten Anzahl an Umschaltzyklen zu stoppen.

7. Vorrichtung nach Anspruch 6, wobei die Steuerung (1048) ferner konfiguriert ist, um die vorbestimmte Anzahl an Umschaltzyklen basierend auf dem gemessenen Druck, der die Okklusion angibt, einzustellen.

8. Vorrichtung nach Anspruch 5, wobei die Steuerung (1048) konfiguriert ist, um das Umschalten nach einer vorbestimmten Menge an Zeit zu stoppen.

9. Vorrichtung nach Anspruch 5, wobei die Steuerung (1048) ferner konfiguriert ist, um einen Alarm zu erzeugen, der einem Benutzer der Infusionspumpe (1020) angibt, dass eine Okklusion in dem Infusionsschlauch (1024) vorliegt, und wobei die Steuerung (1048) konfiguriert ist, um das Umschalten eine Vielzahl von Malen zu wiederholen, bevor der Alarm erzeugt wird.

10. Vorrichtung nach Anspruch 9, wobei die Steuerung (1048) konfiguriert ist, um das Umschalten mindestens zweimal zu wiederholen, bevor der Alarm erzeugt wird.

11. Vorrichtung nach Anspruch 5, wobei die Steuerung (1048) für Folgendes konfiguriert ist:
unter Verwendung des Drucksensors (1032), wiederholtes Messen des Drucks in dem Infusionsschlauch (1024) während des Umschaltens, und
Regulieren des Umschaltens als Reaktion auf das wiederholte Messen des Drucks.

12. Vorrichtung nach Anspruch 11, wobei die Steuerung (1048) konfiguriert ist, um Umschalten zu stoppen, wenn mindestens eine Schwellenwertdruckabnahme aufgrund des Umschaltens durch das wiederholte Messen des Drucks erfasst wird.

13. Vorrichtung nach Anspruch 11, wobei die Steuerung (1048) konfiguriert ist, um das Umschalten zu stoppen, wenn ein Druckwert, der während des wiederholten Messens des Drucks erfasst wird, einen Schwellenwert unterschreitet.

14. Vorrichtung nach Anspruch 13, wobei die Steuerung (1048) konfiguriert ist, um das Umschalten basierend auf einer Menge an Zeit des Umschaltens zu stoppen, selbst wenn der Druckwert, der während des wiederholten Messens des Drucks erfasst wird, den Schwellenwert nicht unterschreitet.

15. Vorrichtung nach Anspruch 13, wobei die Steuerung (1048) konfiguriert ist, um das Umschalten nach einer Anzahl an Umschaltzyklen zu stoppen, wobei die Anzahl kleiner als 100 ist, selbst wenn der Druckwert, der während des wiederholten Messens des Drucks erfasst wird, den Schwellenwert nicht unterschreitet.

## Revendications

1. Appareil destiné à être utilisé avec un tube de perfusion (1024) et un réservoir de fluide (1022), l'appareil comprenant :
une pompe à perfusion (1020) configurée pour être couplée au tube de perfusion (1024) et au réservoir de fluide (1022), la pompe à perfusion (1020) comprenant :
une vanne amont (1026) ;
une vanne aval (1028) ;
une surface de pression (1030) disposée entre les vannes amont et aval (1026, 1028) et configurée pour appuyer sur le tube de perfusion (1024) lorsque la pompe de perfusion (1020) est couplée au tube de perfusion (1024) ;
un capteur de pression (1032) configuré pour mesurer la pression à l'intérieur du tube de perfusion (1024) ; et
un dispositif de commande (1048) configuré pour :
en réponse à la pression mesurée, déterminer qu'il existe une occlusion dans le tube de perfusion (1024) en aval de la vanne aval (1028),
**caractérisé en ce que** ledit dispositif de commande (1048) est en outre configuré pour :
en réponse à la détermination qu'il existe une occlusion, basculer la vanne amont (1026) et la vanne aval (1028), le basculement comprenant :
(a) tandis que la vanne amont (1026) reste fermée, l'isolement d'un segment du tube de perfusion (1024) entre les vannes amont et aval (1026, 1028) en fermant la vanne aval (1028) ;
(b) ensuite, tandis que la vanne aval (1028) reste fermée, la réduction de la pression dans le segment isolé du tube de perfusion (1024) en ouvrant la vanne amont (1026), la pression étant réduite par du fluide de perfusion refluant vers le réservoir lors de l'ouverture de la vanne amont (1026) ;
(c) ensuite, tandis que la vanne aval (1028) reste fermée, la fermeture de la vanne amont (1026) ; et
(d) ensuite, tandis que la vanne amont (1026) reste fermée, la réduction de la pression en aval de la vanne aval (1028) en ouvrant la vanne aval (1028), la pression étant réduite par une partie du fluide de perfusion s'écoulant en amont depuis l'aval de la vanne aval (1028) lors de l'ouverture de la vanne aval (1028).

2. Appareil selon la revendication 1, ledit dispositif de commande (1048) étant configuré pour effectuer le basculement de la vanne amont (1026) et de la vanne aval (1028) tandis que le tube de perfusion (1024) est partiellement comprimé par la surface de pression (1030).

3. Appareil selon l'une quelconque des revendications 1 à 2, ledit capteur de pression (1032) étant positionné entre la vanne amont (1026) et la vanne aval (1028).

4. Appareil selon l'une quelconque des revendications 1 à 3, ledit dispositif de commande 91048) étant en outre configuré pour :
suspendre la génération d'une alerte indiquant l'occlusion en réponse à la détermination qu'il existe une occlusion dans le tube de perfusion (1024) ; et
à la suite du basculement, générer une alerte indiquant à un utilisateur de la pompe à perfusion (1020) qu'il existe une occlusion dans le tube de perfusion (1024).

5. Appareil selon l'une quelconque des revendications 1 à 4, ledit dispositif de commande (1048) étant configuré pour répéter le basculement plusieurs fois, ce qui réduit la pression qui est dans le tube de perfusion (1024) en raison de l'occlusion.

6. Appareil selon la revendication 5, ledit dispositif de commande (1048) étant configuré pour arrêter le basculement après un nombre prédéfini de cycles de basculement.

7. Appareil selon la revendication 6, ledit dispositif de commande (1048) étant en outre configuré pour régler le nombre prédéfini de cycles de basculement en fonction de la pression mesurée indiquant l'occlusion.

8. Appareil selon la revendication 5, ledit dispositif de commande (1048) étant configuré pour arrêter le basculement après une durée prédéfinie.

9. Appareil selon la revendication 5, ledit dispositif de commande (1048) étant en outre configuré pour générer une alerte indiquant à un utilisateur de la pompe à perfusion (1020) qu'il existe une occlusion dans le tube de perfusion (1024), et ledit dispositif de commande (1048) étant configuré pour répéter le basculement plusieurs fois avant de générer l'alerte.

10. Appareil selon la revendication 9, ledit dispositif de commande (1048) étant configuré pour répéter le basculement au moins deux fois avant de générer l'alerte.

11. Appareil selon la revendication 5, ledit dispositif de commande (1048) étant configuré pour :
à l'aide du capteur de pression (1032), mesurer de manière répétée la pression dans le tube de perfusion (1024) pendant le basculement, et
réguler le basculement en réponse à la mesure répétée de la pression.

12. Appareil selon la revendication 11, ledit dispositif de commande (1048) étant configuré pour arrêter le basculement lorsqu'au moins une diminution de pression seuil, due au basculement, est détectée par la mesure répétée de la pression.

13. Appareil selon la revendication 11, ledit dispositif de commande (1048) étant configuré pour arrêter le basculement lorsqu'une valeur de pression détectée lors de la mesure répétée de la pression passe en dessous d'un seuil.

14. Appareil selon la revendication 13, ledit dispositif de commande (1048) étant configuré pour arrêter le basculement en fonction d'une durée du basculement, même si la valeur de pression détectée pendant la mesure répétée de la pression ne passe pas en dessous du seuil.

15. Appareil selon la revendication 13, ledit dispositif de commande (1048) étant configuré pour arrêter le basculement après un certain nombre de cycles de basculement, le nombre étant inférieur à 100, même si la valeur de pression détectée lors de la mesure répétée de la pression ne passe pas en dessous du seuil.
